# EUROPÄISCHE PATENTSCHRIFT

(11) **EP 1 736 772 B1**
(45) Veröffentlichungstag und Bekanntmachung des Hinweises auf die Patenterteilung: **18.05.2016**
(21) Anmeldenummer: 05013396.6
(22) Anmeldetag: 22.06.2005
(51) Int. Cl.: G01N 33/487

(54) **Testvorrichtung mit Testelement-Lagervorrichtung**
Test device with test element storage device
Dispositif de test avec un dispositif de stockage d'éléments d'analyse

(43) Veröffentlichungstag der Anmeldung: 27.12.2006
(73) Patentinhaber: F.Hoffmann-La Roche AG, 4070 Basel (CH); Roche Diagnostics GmbH, 68305 Mannheim (DE)
(72) Erfinder: Bainczyk, Gregor, 68199 Mannheim (DE); Wieder,Herbert, 68259 Mannheim (DE); Eisele, Thomas, 68163 Mannheim (DE)
(74) Vertreter: Herzog, Fiesser & Partner Patentanwälte PartG mbB

(56) Entgegenhaltungen:
- EP-A- 1 488 736
- EP-A- 1 507 143
- WO-A-96/30752
- WO-A-03/083469
- US-A- 6 093 156

## Beschreibung

### Gebiet der Erfindung

Die Erfindung betrifft eine Testvorrichtung, insbesondere eine tragbare Testvorrichtung, zur Bestimmung mindestens einer Analytkonzentration in einer Probe mittels eines Testelements. Weiterhin betrifft die Erfindung ein Testelement zur Bestimmung mindestens einer Analytkonzentration in einer Probe unter Verwendung einer erfindungsgemäßen Testvorrichtung. Derartige Testvorrichtungen und Testelemente werden insbesondere zur Messung von Blutglukosekonzentrationen eingesetzt.

### Stand der Technik

Die Überwachung der Blutglukosekonzentration ist für Diabetiker ein essentieller Bestandteil des Tagesablaufs. Dabei muss die Blutglukosekonzentration schnell und zuverlässig mehrmals am Tag bestimmt werden, um gegebenenfalls entsprechende medizinische Maßnahmen ergreifen zu können. Um den Tagesablauf des Diabetikers nicht mehr als nötig einzuschränken, werden dabei häufig entsprechende mobile Geräte eingesetzt, welche einfach zu transportieren und zu handhaben sein sollten, so dass die Messung der Blutglukosekonzentration beispielsweise am Arbeitsplatz oder auch in der Freizeit erfolgen kann.

Derzeit sind verschiedene mobile Geräte auf dem Markt, welche teilweise nach unterschiedlichen Messmethoden funktionieren. Dabei kommen verschiedene Diagnoseverfahren zum Einsatz, beispielsweise optische oder auch elektrochemische Messverfahren.

EP 1 488 736 A1 beschreibt einen Container mit einem drehbaren Deckel zum Auslesen und Handhaben von diagnostischen Reagenzien in Streifenform, umfassend einen Körperabschnitt, einen Deckelabschnitt ein kontinuierliches Band, eine Reagenzien-Messvorrichtung und eine Lagervorrichtung. Der Deckelabschnitt ist an dem Körperabschnitt befestigt und ist eingerichtet, um von einer geschlossenen Position in eine offene Position zu rotieren.

WO 96/30752 A1 beschreibt eine Vorrichtung zur Überwachung eines Glucoselevels. Die Vorrichtung weist ein Gehäuse sowie einen Deckel auf. Der Deckel ist eingerichtet, um eine Basis des Gehäuses zu bedecken oder zu freizugeben.

EP 1 507 143 A1 beschreibt eine analytische Instrumentenpackung mit einem analytischen Instrument, welches zwischen einer oder mehreren Siegelschichten eingebettet ist. Die analytische Instrumentenpackung umfasst einen Stopper-Abschnitt zum Halten des analytischen Instruments, wobei das analytische Instrument aus den Siegelschichten hervorsteht.

Ein Beispiel eines häufig eingesetzten Messverfahrens nutzt eine spezielle Art elektrochemischer Teststreifen. Diese Teststreifen sind beispielsweise so aufgebaut, dass eine vorgegebene Blutmenge über ein Kapillarensystem auf dem Teststreifen zu einem Elektrodensystem geführt wird. Für moderne Teststreifen genügt dabei eine Blutmenge von ca. 1,5 µl, teilweise auch Blutmengen unter 1 µl. Bei dem Elektrodensystem kann es sich z. B. um Goldelektroden handeln, welche mit einer Beschichtung versehen sind. Die Beschichtung enthält zumeist verschiedene Enzyme und so genannte Mediatoren und bewirkt, dass sich innerhalb der Probe an den Elektroden Ladungsträger (beispielsweise in Form von Redox-Molekülen) bilden, deren Konzentration abhängig ist von der Blutglukosekonzentration. Die Konzentration dieser Ladungsträger kann mittels der Goldelektroden und einem geeigneten Messsystem, beispielsweise mittels einer Strom-Spannungs-Messung bestimmt werden, so dass daraus schließlich auf die Blutglukosekonzentration zurückgerechnet werden kann. Ein Beispiel derartiger elektrochemischer Teststreifen ist in US 5,286,362 dargestellt.

Alternativ zu dem beschriebenen elektrochemischen Messverfahren lassen sich auch andere Messprinzipien einsetzen. So beschreibt beispielsweise die WO 01/48461 einen Lichtleiter-Teststreifen zur Untersuchung einer Probe, insbesondere einer Körperflüssigkeit, bei dem ein Reagenzsystem bei Reaktion mit der Probe zu einer charakteristischen, optisch messbaren Änderung in einer Detektionszone führt. Über Lichtleiter, die in den Teststreifen eingelassen sind, kann diese Änderung von einem Auswertegerät ausgewertet werden.

Die Teststreifen bilden somit ein wesentliches Element portabler Diagnosesysteme. Typischerweise werden von einem Diabetiker ca. 5 bis 7 derartiger Teststreifen pro Tag benötigt. Essentiell ist dabei, dass die Teststreifen sauber und trocken aufbewahrt werden, um nicht durch eine entsprechende Verschmutzung bzw. Einwirkung von Feuchtigkeit die Messung der Blutglukosekonzentration zu verfälschen.

Zu diesem Zweck werden die Teststreifen üblicherweise in entsprechenden Behältnissen aufbewahrt, um anschließend vom Benutzer für eine Messung dem Teststreifenbehältnis entnommen und in ein entsprechendes Messgerät eingegeben zu werden. Derartige Messgeräte, beispielsweise Messgeräte für eine elektrochemische Bestimmung der Blutglukosekonzentration, sind dem Fachmann bekannt und werden beispielsweise in US 2002/0170823 A1 beschrieben.

Die aus dem Stand der Technik bekannten Messsysteme, bei welchen eine Blutglukosekonzentration mittels eines einzelnen Teststreifens bestimmt wird (Einzelstreifensysteme), wobei jeweils ein einzelner Teststreifen für eine Messung in das Gerät eingegeben werden muss, weisen jedoch in der Praxis zahlreiche Nachteile auf. So erfordern derartige Systeme zahlreiche Handhabungsschritte durch den Nutzer bzw. Patienten. Ein Teststreifen muss einer entsprechenden Aufbewahrungsvorrichtung (z. B. einer Teststreifendose) entnommen werden und manuell in ein Messgerät eingefügt werden. So muss ein entsprechender Teststreifen für derartige Einzelstreifensysteme für eine sichere Handhabung durch einen Patienten, insbesondere durch ältere Patienten oder Kinder, vergleichsweise groß ausgestaltet sein, um zu verhindern, dass der Teststreifen den Fingern des Patienten entgleitet und für eine Messung unbrauchbar wird. Eine erhöhte Größe der Teststreifen trägt jedoch dazu bei, dass der Platzbedarf der Messsysteme steigt und weniger Teststreifen in einem entsprechenden Behältnis untergebracht werden können. Außerdem steigen durch diesen erhöhten Platzbedarf die Materialkosten der Messsysteme.

Weiterhin ist die manuelle Handhabung der Teststreifen mit dem Nachteil einer erhöhten Verschmutzungsgefahr der Teststreifen verbunden. So kann eine manuelle Entnahme der Teststreifen aus einem Teststreifenbehältnis zu einer Beaufschlagung der Teststreifen mit Fingerschweiß oder anderen Verschmutzungen führen, welche eine entsprechende Messung beeinträchtigen könnten. Ein weiterer erheblicher Nachteil besteht darin, dass bei jedem Öffnen des Teststreifenbehältnisses die im Teststreifenbehältnis verbleibenden Teststreifen mit Luftfeuchtigkeit beaufschlagt werden, so dass später verwendete Teststreifen aus einem Teststreifenbehältnis möglicherweise andere Eigenschaften aufweisen als die zuerst entnommenen Teststreifen.

Um die Nachteile derartiger Einzelstreifensysteme zu vermeiden, wurden integrierte Systeme entwickelt, insbesondere Systeme, bei welchen neben einer Messvorrichtung auch ein Magazin für Teststreifen integriert ist. Die Systeme können derart ausgestaltet sein, dass jeweils ein Teststreifen für eine Messung aus dem Magazin in eine Messposition ausgegeben wird. Nach Beaufschlagung mit der entsprechenden Probe, beispielsweise einem Blutstropfen, wird dann unmittelbar die Messung durchgeführt.

Ein Beispiel eines derartigen integrierten Systems ist in der WO 02/18940 A2 beschrieben. Die WO 02/18940 A2 offenbart ein Testgerät zur Messung einer Analytkonzentration in einem Fluid, welches eine Mehrzahl von gestapelten Sensoren aufweist, die in einem Gehäuse mit einer Öffnung aufgenommen sind. In einer Messposition können die Sensoren elektrisch kontaktiert und ausgewertet werden. In der Öffnung des Gehäuses ist ein Transportelement rotierbar aufgenommen, und das Testgerät weist ein Federelement auf, welches jeweils einen einzelnen Sensor in eine Aussparung des Transportelements transferiert. Wird das Transportelement gedreht, so wird jeweils ein Sensor zur Messposition befördert.

Integrierte Systeme weisen gegenüber herkömmlichen Einzelstreifensystemen erhebliche Vorteile auf. So wird die Anzahl der erforderlichen Handhabungsschritte für eine Messung reduziert, und es lassen sich auch kleine, kostengünstige Testelemente einsetzen. Auch wird die Beaufschlagung der Testelemente, insbesondere der Teststreifen, mit Feuchtigkeit und Verschmutzungen erheblich reduziert.

Die aus dem Stand der Technik bekannten integrierten Systeme weisen jedoch auch Nachteile auf, insbesondere Nachteile, welche den Einsatz als portable Geräte stören und die Akzeptanz durch einen Patienten verringern. Ein wesentlicher Nachteil besteht darin, dass die Integration der Systeme zu vergleichsweise großen Baugrößen der Geräte führt.. So sind für viele integrierte Systeme beispielsweise vergleichsweise voluminöse Federsysteme erforderlich, welche einen entsprechenden Transport der Teststreifensysteme ermöglichen. Zudem werden integrierte Systeme häufig elektromotorisch angetrieben, was zu einem zusätzlichen Platzbedarf führt. Integrierte Systeme, welche nicht elektromotorisch angetrieben werden, erfordern hingegen zusätzliche Spannvorrichtungen, was wiederum eine Handhabung insbesondere durch ältere Patienten oder Kinder in vielen Fällen stört. Zudem werden durch elektromotorische Antriebe oder komplexe manuelle Antriebe die Herstellkosten der Systeme stark erhöht. Elektrisch angetriebene Systeme weisen zusätzlich den Nachteil auf, dass für die Elektromotoren leistungsfähige Batterien erforderlich sind, welche teuer sind und oft gewechselt werden müssen. Diese Batterien führen außerdem zu einer zusätzlichen Erhöhung des Bauvolumens. Ein weiterer Nachteil der komplex ausgestalteten integrierten Systeme, welche aus dem Stand der Technik bekannt sind, besteht darin, dass derartige Systeme häufig empfindliche Bauteile auf ihren Oberflächen aufweisen, welche beispielsweise bei einem Transport in einer Tasche leicht mechanisch beschädigt werden können, welche ein Eindringen von Feuchtigkeit in die Geräte ermöglichen, und/oder welche durch eine Verschmutzung in ihrer Funktionalität beeinträchtigt werden können.

### Aufgabe der Erfindung

Aufgabe der vorliegenden Erfindung ist es, eine Testvorrichtung zur Bestimmung mindestens einer Analytkonzentration in einer Probe, insbesondere einer flüssigen Probe, mittels mindestens eines Testelements bereitzustellen, welche die Nachteile der aus dem Stand der Technik bekannten Testvorrichtungen vermeidet. Weiterhin soll ein Magazin mit Testelementen bereitgestellt werden, welches in der erfindungsgemäßen Testvorrichtung eingesetzt werden kann.

### Beschreibung der Erfindung

Diese Aufgabe wird durch die Erfindung mit den Merkmalen des unabhängigen Anspruchs gelöst. Vorteilhafte Weiterbildungen der Erfindung sind in den Unteransprüchen gekennzeichnet. Der Wortlaut sämtlicher Ansprüche wird hiermit durch Bezugnahme zum Inhalt dieser Beschreibung gemacht.

Es wird eine Testvorrichtung zur Bestimmung mindestens einer Analytkonzentration in einer Probe mittels eines Testelements vorgeschlagen. Insbesondere kann es sich dabei um eine Glukosemessung, insbesondere eine Blutglukosemessung, und/oder eine Cholesterinmessung und/oder eine Koagulationsmessung handeln. Alternativ oder zusätzlich können jedoch auch andere Analytkonzentrationen ermittelt werden oder entsprechende andere Analysen durchgeführt werden, beispielsweise pH-Messungen oder ähnliche chemische Analysen oder Messungen. Auch können beispielsweise immunologische Messungen oder ähnliche Messungen mit der Testvorrichtung durchgeführt werden. Bei der Probe soll es sich insbesondere um eine flüssige Probe, beispielsweise Blut, Urin, Speichel oder Stuhl, handeln. Es sind jedoch auch andere Arten von Proben denkbar, beispielsweise gasförmige Proben. Weiterhin kann anstelle einer Analytkonzentration auch lediglich auf die Anwesenheit oder Nicht-Anwesenheit eines Analyten geschlossen werden, was erfindungsgemäß mit umfasst sein soll.

Die Testvorrichtung weist ein Gehäuse auf, welches einen geschlossenen Zustand und einen geöffneten Zustand aufweist. Das Gehäuse kann insbesondere derart ausgestaltet sein, dass das Gehäuse manuell durch einen Benutzer zu öffnen ist. Insbesondere kann das Gehäuse aufschiebbar oder aufklappbar ausgestaltet sein. Beispielsweise kann das Gehäuse eine im Wesentliche quaderförmige Form aufweisen mit einer Klappachse, wobei das Gehäuse entlang der Klappachse aufgeklappt werden kann, so dass ein Gehäuseunterteil und ein Gehäuseoberteil im aufgeklappten Zustand winklig zueinander stehen, beispielsweise in einem rechten Winkel. Im geschlossenen Zustand liegen Gehäuseunterteil und Gehäuseoberteil aufeinander, wobei jeweils die Flächen, entlang derer Gehäuseunterteil und Gehäuseoberteil aneinander anliegen, von außen nicht mehr zugänglich sind. Dies kann beispielsweise so erfolgen, dass die Flächen, entlang derer Gehäuseunterteil und Gehäuseoberteil aneinander anliegen, im geschlossenen Zustand des Gehäuses durch das Gehäuse abgedichtet sind, beispielsweise um diese Flächen und Elemente, die auf und/oder in diesen Flächen angeordnet sind (z. B. Displays, Bedienelemente, Teststreifen etc.), vor Umwelteinflüssen, z. B. Luftfeuchtigkeit, Schmutz oder mechanischen Einwirkungen, zu schützen. Derartige Systeme sind aus dem Stand der Technik beispielsweise für Mobiltelefone bekannt. Es sind jedoch auch andere erfindungsgemäße Öffnungsvorrichtungen denkbar, beispielsweise Schiebevorrichtungen.

Das erfindungsgemäße Merkmal, dass das Gehäuse geöffnet werden kann, stellt einen erheblichen Vorteil gegenüber bekannten Systemen dar. Insbesondere die Weiterbildung, bei welcher das Gehäuse aufgeklappt werden kann, ermöglicht es, empfindliche Komponenten, welche einem Benutzer zugänglich sein sollen, in einem Bereich des Gehäuses unterzubringen, welcher nur im aufgeklappten bzw. geöffneten Zustand des Gehäuses zugänglich ist.

Weiterhin weist die Testvorrichtung eine Lagervorrichtung zur Aufnahme von Testelementen auf, wobei die Lagervorrichtung mindestens eine Lagerposition für Testelemente aufweist. Beispielsweise kann die Lagervorrichtung derart ausgestaltet sein, dass mehrere Testelemente vorgesehen sind, welche jeweils einzeln in jeweils einer Kavität der Lagervorrichtung gelagert sind. Im Gegensatz zu vielen aus dem Stand der Technik bekannten Vorrichtungen, beispielsweise der aus der WO 02/18940 A2 bekannten Vorrichtung, wird bei dieser Ausgestaltung der Lagervorrichtung sichergestellt, dass bei Entnahme eines Testelements die übrigen in der Lagervorrichtung verbleibenden Testelemente unbeeinflusst bleiben. Zusätzlich kann die Lagervorrichtung ein Trockenmittel aufweisen, um eine trockene und saubere Lagerung der Testelemente zu gewährleisten. So kann beispielsweise ein Trockenmittel in eine oder mehrere Kavitäten der Lagervorrichtung eingebracht sein. Alternativ oder zusätzlich kann auch ein Trockenmittel in einem sonstigen Hohlraum oder einer Wand der Lagervorrichtung, insbesondere des Magazins, eingebracht sein. Weiterhin können alternativ oder zusätzlich auch die Testelemente selbst Trockenmittel aufweisen, also beispielsweise Abschnitte, welche Feuchtigkeit aufnehmen können.

Weiterhin weist die erfindungsgemäße Testvorrichtung eine Messvorrichtung auf. Die Messvorrichtung ist derart ausgestaltet, dass mittels der Testelemente die mindestens eine Analytkonzentration bestimmt werden kann. Derartige Messvorrichtungen sind dem Fachmann bekannt und sollen daher an dieser Stelle nicht weiter beschrieben werden. Insbesondere hängt die Art der Messvorrichtung von der Art des verwendeten Testelements ab, dessen mögliche Ausgestaltung unten näher beschrieben werden soll. Beispielsweise kann die mindestens eine Messposition Kontakte zur Kontaktierung des mindestens einen Testelements aufweisen.

Weiterhin weist die Testvorrichtung eine Ausgabevorrichtung auf. Die Ausgabevorrichtung ist mit Mitteln ausgestattet, welche eine Beförderung der Testelemente von der mindestens einen Lagerposition in mindestens eine Messposition bei Öffnen des Gehäuses ermöglichen. Unter einem "Öffnen" des Gehäuses kann dabei insbesondere ein Zugänglichmachen von im geschlossenen Zustand verborgenen Komponenten, beispielsweise von Flächen, entlang derer Gehäuseunterteil und Gehäuseoberteil aneinander anliegen, sowie von auf diesen Flächen angeordneten Bedienelementen (Displays, Bedienknöpfen, Teststreifenmagazin) verstanden werden. Beispielsweise kann hierunter ein Aufschieben oder Aufklappen des Gehäuses verstanden werden. Vorzugsweise wird dabei auch eine Teststreifenausgabe zugänglich, beispielsweise indem beim Öffnen des Gehäuses ein Ausgabeschlitz oder ähnliches freigegeben wird. Auch eine andere Art von "Zugänglichmachen von Komponenten" kann unter dem Begriff "Öffnen" subsumiert werden.

Insbesondere kann die Beförderung des mindestens einen Testelements von der mindestens einen Lagerposition in die mindestens eine Messposition durch das Öffnen des Gehäuses ausgelöst und/oder bewirkt werden. Beispielsweise kann der Kraftaufwand, welcher für einen Benutzer zum Öffnen des Gehäuses erforderlich ist, genutzt werden, um die Beförderung des Testelements zu bewerkstelligen. Beispielsweise kann die Kraft, welche beim Öffnen des Gehäuses auf das Gehäuse ausgeübt wird, mittels einer entsprechenden mechanischen Vorrichtung, beispielsweise einer Hebelvorrichtung, auf das mindestens eine Testelement übertragen werden.

Dabei ist es besonders vorteilhaft, wenn die Beförderung des mindestens einen Testelemente durch die Ausgabevorrichtung eine Drehbewegung des Testelements um eine Drehachse umfasst. Insbesondere kann das mindestens eine Testelement eine Längserstreckung haben, beispielsweise eine Längsachse eines Teststreifens, wobei vorteilhafterweise die Drehung des Testelements bei der Beförderung von der mindestens einen Lagerposition in die mindestens eine Messposition um eine Drehachse senkrecht zur Längsachse des Testelements erfolgt. So kann die Ausgabevorrichtung eine derartige Drehachse aufweisen. Beispielsweise kann diese Drehachse das mindestens eine Testelement durchsetzen.

Alternativ oder zusätzlich kann die Beförderung des Testelements von der mindestens einen Lagerposition in die mindestens eine Messposition auch durch eine anders ausgestaltete Bewegung erfolgen, beispielsweise eine Translationsbewegung. So kann beispielsweise beim Öffnen des Gehäuses ein Teststreifen in eine Messposition geschoben werden. Auch eine Kombination von Bewegungsarten, beispielsweise eine Translations-Rotationsbewegung, ist möglich.

Die erfindungsgemäße Testvorrichtung weist gegenüber anderen integrierten Testvorrichtungen, welche aus dem Stand der Technik bekannt sind, zahlreiche Vorteile auf. Insbesondere die Öffnungsfunktion des Gehäuses, beispielsweise das Aufklappen des Gehäuses, ermöglicht es, transportable Testvorrichtungen zu gestalten, welche sich durch äußerste Robustheit auszeichnen. So kann das Gehäuse derart ausgestaltet sein, dass im geschlossenen Zustand des Gehäuses von außen lediglich unempfindliche Komponenten der Testvorrichtung zugänglich sind. Beispielsweise kann das Gehäuse auf seiner Außenseite mit einer glatten, schmutzabweisenden Oberfläche ausgestaltet sein, welche ein Eindringen von Schmutz und Feuchtigkeit in die Testvorrichtung verhindert. Alternativ können jedoch auch einzelne Bedienelemente auf der äußeren Oberfläche des Gehäuses angeordnet sein, beispielsweise einzelne Bedienknöpfe oder Anzeigeelemente. Von Vorteil ist es jedoch, wenn die wesentlichen und insbesondere die empfindlichen Funktionselemente der Testvorrichtung erst im geöffneten Zustand des Gehäuses zugänglich sind. Insbesondere kann die Testvorrichtung derart ausgestaltet sein, dass Bedienknöpfe, Bedientasten, Anzeigeelemente und weitere Funktionselemente der Testvorrichtung im aufgeklappten Zustand zugänglich sind.

Die Testvorrichtung bietet weiterhin einen hohen Grad an Diskretion, da die Testvorrichtung flach ausgestaltet werden kann und somit unauffällig getragen werden kann. Ein störendes Motor- oder Getriebegeräusch tritt bei der Benutzung der Testvorrichtung nicht auf, da auf einen Elektromotor verzichtet werden kann. Die Systemkosten der Testvorrichtung sind vergleichsweise gering, da auf kostenträchtige Bauteile, wie z. B. Motoren, große Batterien etc. verzichtet werden kann. Auch die Materialkosten, insbesondere für Verbrauchsmaterial, sind vergleichsweise gering, da kleinere Testelemente eingesetzt werden können.

Weiterhin ist durch die erfindungsgemäße Ausgestaltung der Testvorrichtung die Handhabungssicherheit gegenüber aus dem Stand der Technik bekannten integrierten Systemen deutlich erhöht. Für die Vorbereitung einer Bestimmung einer Analytkonzentration, beispielsweise einer Blutglukosekonzentrationsmessung, ist nunmehr lediglich ein Öffnen, beispielsweise ein Aufklappen, der Testvorrichtung erforderlich. Ein Spannen einer Transportvorrichtung oder ein Betätigen eines Elektromotors durch einen entsprechenden Knopf ist nicht mehr erforderlich. Somit ist insbesondere sichergestellt, dass auch Patienten mit eingeschränkten motorischen Fähigkeiten, insbesondere ältere Patienten oder Kinder, die Testvorrichtung sicher handhaben können.

Ein weiterer erheblicher Vorteil der erfindungsgemäßen Ausgestaltung der Testvorrichtung, besteht darin, dass das Bauvolumen derartiger Testvorrichtungen gegenüber bekannten Testvorrichtungen erheblich verringert werden kann. Für eine erfindungsgemäße Testvorrichtung sind weder aufwändige und kostenträchtige Elektromotoren mit voluminösen und teuren Batterien erforderlich, noch ist eine komplexe und voluminöse Spannvorrichtung bzw. Transportvorrichtung für Testelemente nötig. Insbesondere die Ausgestaltung, bei welcher ein Testelement um eine Achse rotiert wird, um das Testelement von der Lagerposition in die Messposition zu bringen, ermöglicht eine platzsparende Realisierung der Testvorrichtung. Insgesamt ist also festzustellen, dass die erfindungsgemäße Testvorrichtung sich in hervorragender Weise für den Einsatz als robustes, einfach zu handhabendes und kostengünstiges mobiles Gerät handelt, welches auch für Patienten mit eingeschränkten motorischen Fähigkeiten leicht handhabbar ist. Dies fördert den so genannten "Home-Care-Gedanken" und verringert Kosten des Gesundheitssystems.

Die Lagervorrichtung weist ein Magazin auf. Beispielsweise kann es sich dabei um ein auswechselbares Magazin handeln, wie es beispielsweise im Rahmen des nebengeordneten Anspruchs 13 separat beansprucht wird. Derartige Magazine können beispielsweise vom Patienten über eine Apotheke oder einen medizinischen Versandhandel bezogen und jeweils in die Testvorrichtung eingesetzt werden. Dabei kann es sich um Einwegmagazine oder auch um wiederverwendbare Magazine handeln. In ein auswechselbares Magazin kann beispielsweise auch ein elektronischer Speicher oder eine andere Art von Informationsträger integriert sein, so dass beim Einlegen eines neuen Magazins in die Testvorrichtung auch chargenspezifische Informationen über die in dem Magazin enthaltenen Testelemente in die Testvorrichtung übertragen werden können. Derartige so genannte "Non-evident-coding"-Systeme sind aus dem Stand der Technik bekannt.

Von besonderem Vorteil ist es, wenn die Lagervorrichtung, beispielsweise das Magazin, eine perforierbare Membran, beispielsweise eine Folie, zum Schutz des mindestens einen Testelements gegen Luftfeuchtigkeit und Schmutz (Versiegelung) aufweist. Diese Weiterbildung der Erfindung ist insbesondere von Vorteil in Kombination mit der erfindungsgemäßen Ausgestaltung der Erfindung, bei welcher die Lagervorrichtung für jedes Testelement eine separate Kavität aufweist. So kann jede Kavität durch eine derartige perforierbare Membran abgeschlossen sein. Beispielsweise kann die Ausgabevorrichtung und/oder das mindestens eine Testelement derart ausgestaltet sein, dass bei der Beförderung des mindestens einen Testelements von der mindestens einen Lagerposition in die mindestens eine Messposition beim Öffnen des Gehäuses die Membran perforiert wird. Beispielsweise kann zu diesem Zweck das mindestens eine Testelement eine scharfe Kante oder Ecke aufweisen. Insbesondere kann die Perforation durch eine Drehbewegung des mindestens einen Testelements erfolgen, bei welcher beispielsweise eine scharfe Ecke des mindestens einen Testelements gegen die Membran gepresst wird, so lange, bis diese perforiert wird und das Testelement durch die Membran hindurch in die Messposition befördert werden kann.

Weiterhin kann die Testvorrichtung erfindungsgemäß auch dahingehend weitergebildet werden, dass beim Schließen des Gehäuses das mindestens eine Testelement wieder aus der mindestens einen Messposition zurück in die mindestens eine Lagerposition befördert wird. Insbesondere kann diese Beförderung ebenfalls durch die Ausgabevorrichtung erfolgen, welche entsprechende Mittel zu dieser Beförderung aufweisen kann. Beispielsweise kann es sich bei dieser Beförderung wiederum um eine Drehung des mindestens einen Testelements handeln. Diese Weiterbildung der Erfindung gewährleistet, dass benutzte Testelemente nicht entnommen werden müssen, sondern beispielsweise in die Lagervorrichtung zurück überführt werden, wo diese insbesondere hygienisch aufbewahrt sind. Eine separate Entsorgung jedes einzelnen Testelements ist nicht erforderlich. Auch die Diskretion der Benutzung wird damit erhöht.

Die Erfindung kann auch dahingehend weitergebildet werden, dass ein Entkopplungsmechanismus vorgesehen ist, welcher die Ausgabevorrichtung vom Öffnen der Testvorrichtung auf Wunsch eines Benutzers entkoppelt. Dies kann beispielsweise durch ein einfaches Entkoppeln von Getrieben (z. B. eines mit dem Gehäuse verbundenen Zahnrades von einem mit der Ausgabevorrichtung verbundenem Zahnrad) erfolgen, auf eine Weise, die dem Fachmann bekannt ist. So kann beispielsweise das Gehäuse geöffnet werden, ohne dass ein Testelement ausgegeben wird, wenn ein Benutzer dies wünscht und vorgibt (beispielsweise durch Drücken eines Entkopplungsknopfes). Damit kann ein Benutzer beispielsweise das Gehäuse öffnen, um z. B. elektronische Messergebnisse aus einem Speicher der Testvorrichtung auszulesen, ohne dass ein Testelement verschwendet wird. Alternativ kann das Gehäuse auch so ausgestaltet sein, dass dieses nur unter Ausgabe eines Testelements geöffnet werden kann.

Grundsätzlich kann die Testvorrichtung derart ausgestaltet sein, dass beim Öffnen der Testvorrichtung ein einzelnes Testelement in eine Messposition befördert wird. Alternativ können jedoch auch mehrere Testelemente gleichzeitig in derartige Messpositionen befördert werden. Bevorzugt ist jedoch die Verwendung eines einzelnen Testelements.

Um zu gewährleisten, dass bei jedem Öffnen des Gehäuses jeweils ein frisches, d. h. bislang unbenutztes Testelement eingesetzt wird, kann die Testvorrichtung zusätzlich eine Auswahlvorrichtung aufweisen. Diese Auswahlvorrichtung kann derart ausgestaltet sein, dass bei jedem Öffnen des Gehäuses ein bislang unbenutztes Testelement ausgewählt wird. Beispielsweise können Testelemente parallel zueinander in einer Reihe, beispielsweise in einzelnen Kavitäten, angeordnet sein, wobei die Auswahlvorrichtung bei jedem Öffnen des Gehäuses jeweils einen Schritt in Richtung des nächsten, bislang unbenutzten Testelements ausführt. Beispielsweise kann die Auswahlvorrichtung dabei jeweils um eine Position einer Kavität weiterbewegt werden.

Grundsätzlich lässt sich die erfindungsgemäße Testvorrichtung mit einer Vielzahl von streifenförmigen Testelementen und Testprinzipien einsetzen. Derartige Testprinzipien sind dem Fachmann aus dem Stand der Technik bekannt. So können beispielsweise optische Messmethoden eingesetzt werden, beispielsweise zur optischen Bestimmung eines Cholesteringehalts in Blut oder Urin, was z. B. aus DE 698 15 207 T2 bekannt ist. Alternativ oder zusätzlich können jedoch auch elektrochemische Teststreifen eingesetzt werden, wie oben im Zusammenhang mit Blutglukosekonzentrationsmessungen bereits beschrieben wurde und was beispielsweise aus US 5,286,362 bekannt ist.

Im Zusammenhang mit der erfindungsgemäßen Testvorrichtung wird ein Testelement vorgeschlagen, welches mit der Ausgabevorrichtung der Testvorrichtung zusammenwirken kann. Zu diesem Zweck weist das Testelement mindestens eine Verbindungsvorrichtung zur Verbindung des Testelements mit der Ausgabevorrichtung auf. Beispielsweise kann es sich bei dieser Verbindungsvorrichtung um einen Haken handeln, welcher in eine entsprechende Aussparung der Ausgabevorrichtung eingreift. Besonders bevorzugt ist es jedoch, wenn das Testelement eine Öffnung zur Durchführung einer Achse der Ausgabevorrichtung aufweist. Diese Öffnung kann beispielsweise eine runde oder eckige Öffnung im Innenbereich des Testelements sein, oder es kann sich bei dieser Öffnung auch um eine am Rand des Testelements angeordnete Öffnung, beispielsweise eine Aussparung am Rand des Testelements (vorzugsweise des Teststreifens) handeln. Auf diese Weise kann mittels der Achse das Testelement gedreht werden, um dieses von der Lagerposition in die Messposition zu überführen.

Weiterhin kann das Testelement derart ausgestaltet sein, dass dieses ein Kapillarsystem zur Beförderung einer flüssigen Probe von einer Applikationsstelle zu einer Messstelle aufweist. Beispielsweise kann das Testelement derart ausgestaltet sein, dass dieses eine Öffnung zur Durchfiihrung einer Achse aufweist, welche beispielsweise in der Mitte des Testelements oder am Rand des Testelements angeordnet ist. Weiterhin kann das Testelement an gegenüberliegenden Enden einer Applikationsstelle und Elektrodenkontakte aufweisen. Insbesondere kann dann die Testvorrichtung derart ausgestaltet sein, dass das Testelement durch die Ausgabevorrichtung beim Öffnen des Gehäuses von der Lagerposition in die Messposition gedreht wird, wodurch die Applikationsstelle für den Benutzer zugänglich wird und wobei gleichzeitig die Messelektroden des Testelements in die Kontakte der Testvorrichtung eingreifen. Somit wird beim Befördern des Testelements in die Messposition das Testelement automatisch elektrisch kontaktiert.

Die erfindungsgemäße Testvorrichtung und das Testelement können von einem Patienten beispielsweise auf die folgende Weise genutzt werden, wobei die im Folgenden beschriebenen Verfahrensschritte nicht notwendigerweise auf die dargestellte Reihenfolge beschränkt sind. So trägt der Patient die Testvorrichtung im geschlossenen Zustand in einem entsprechenden Transportbehältnis oder in der Tasche. Zur Durchführung einer Messung, beispielsweise für eine Blutglukosekonzentrationsmessung, entnimmt der Patient die Testvorrichtung der Tasche und öffnet das Gehäuse der Testvornchtung. Beispielsweise kann dieses Öffnen durch einfaches Aufklappen erfolgen, oder es kann zum Öffnen die zusätzliche Betätigung eines Bedienelements erforderlich sein. Beispielsweise kann die Testvorrichtung einen Öffnungsknopf aufweisen, welcher zum Öffnen der Testvorrichtung betätigt werden muss. Auf diese Weise kann ein unbeabsichtigtes Öffnen der Testvorrichtung verhindert werden.

Durch das Öffnen der Testvorrichtung wird automatisch ein Testelement, beispielsweise ein Teststreifen, in eine Messposition befördert. Insbesondere kann beispielsweise das Testelement dabei elektrisch kontaktiert werden. Weiterhin kann die Testvorrichtung auch derart ausgestaltet sein, dass beim Öffnen des Gehäuses automatisch die Messvorrichtung gestartet wird. Dieses Starten der Messvorrichtung kann beispielsweise ein Anschalten eines Computers, beispielsweise eines in der Messvorrichtung integrierten Mikrocomputers, umfassen. Weiterhin kann dem Benutzer auf einem Anzeigeelement, beispielsweise einem Display, automatisch ein entsprechendes Menü zur Verfügung gestellt werden. Das Gehäuse kann derart ausgestaltet sein, dass beim Schließen der Testvorrichtung der Computer automatisch wieder abgeschaltet wird.

In der Zwischenzeit hat der Patient eine entsprechende Probe bereitgestellt. Beispielsweise kann dieses Bereitstellen einer Probe die Erzeugung eines Blutstropfens, beispielsweise mittels eines dem Fachmann bekannten Lanzettensystems, umfassen. Der Patient appliziert die Probe auf das Testelement. Anschließend wird, automatisch oder durch den Patienten initiiert (z. B. durch Drücken eines entsprechenden Messknopfes), die Analytkonzentration in der Probe bestimmt. Die Analytkonzentration kann beispielsweise auf einem Anzeigeelement (z. B. einem Display) dargestellt und/oder in einem Datenspeicher eines Mikrocomputers abgespeichert werden. Alternativ oder zusätzlich kann die Analytkonzentration beispielsweise in eine Datenbank eingetragen werden und entsprechend weiter einer Datenaufbereitung unterzogen werden.

Anschließend verschließt der Patient das Gehäuse der Testvorrichtung wieder, wobei beispielsweise das benutzte Testelement wieder zurück in die Lagervorrichtung befördert wird. Die Testvorrichtung kann dann wieder in einer entsprechenden Transportvorrichtung verstaut werden, beispielsweise in einer Tasche. Alternativ oder zusätzlich können die gewonnenen Daten, beispielsweise Daten, welche die Analytkonzentration umfassen, an andere Auswertungsgeräte, beispielsweise andere Computersysteme, übermittelt werden oder einem Arzt für eine weitere Auswertung zur Verfügung gestellt werden.

### Ausführungsbeispiele

Im Folgenden wird die Erfindung anhand von Ausführungsbeispielen näher beschrieben. Die Erfindung ist jedoch nicht auf die dargestellten Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigt:
- Figur 1A: ein erstes, bevorzugtes Ausführungsbeispiel einer erfindungsgemäßen Testvorrichtung in geschlossenem Zustand;
- Figur 1B: die Testvorrichtung gemäß Figur 1A während des Öffnens;
- Figur 1C: die Testvorrichtung gemäß Figur 1A in geöffnetem Zustand;
- Figur 2A: ein zweites Ausführungsbeispiel einer Testvorrichtung in geschlossenem Zustand;
- Figur 2B: die Testvorrichtung gemäß Figur 2A in geöffnetem Zustand;
- Figur 3: ein Ausführungsbeispiel eines Teststreifenmagazins zum Einsatz in einer erfindungsgemäßen Testvorrichtung;
- Figur 4A: ein erstes Ausführungsbeispiel eines Teststreifens;
- Figur 4B: ein zweites Ausführungsbeispiel eines Teststreifens;
- Figuren 5A bis 5E: ein Ausführungsbeispiel eines schematischen Verfahrensablaufs der Beförderung eines Teststreifens von einer Lagerposition in eine Messposition beim Öffnen einer Testvorrichtung; und
- Figuren 6A bis 6D: ein Ausführungsbeispiel einer Auswahlvorrichtung zur Auswahl eines Testsstreifens aus dem Magazin.

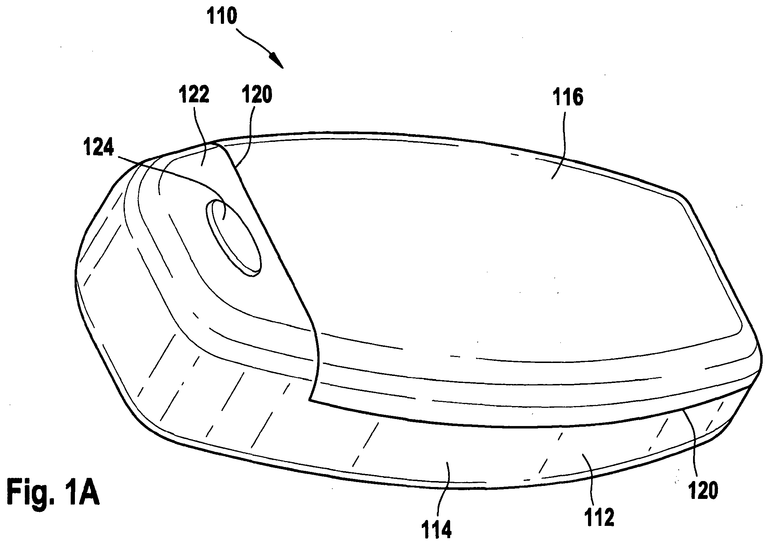

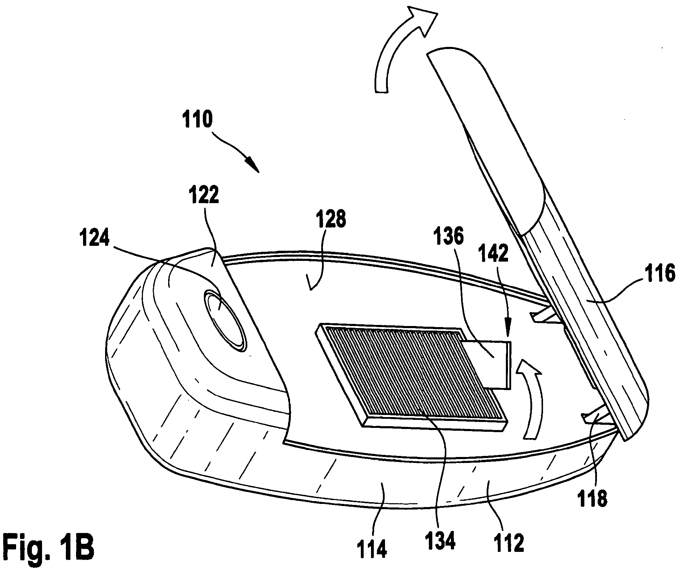

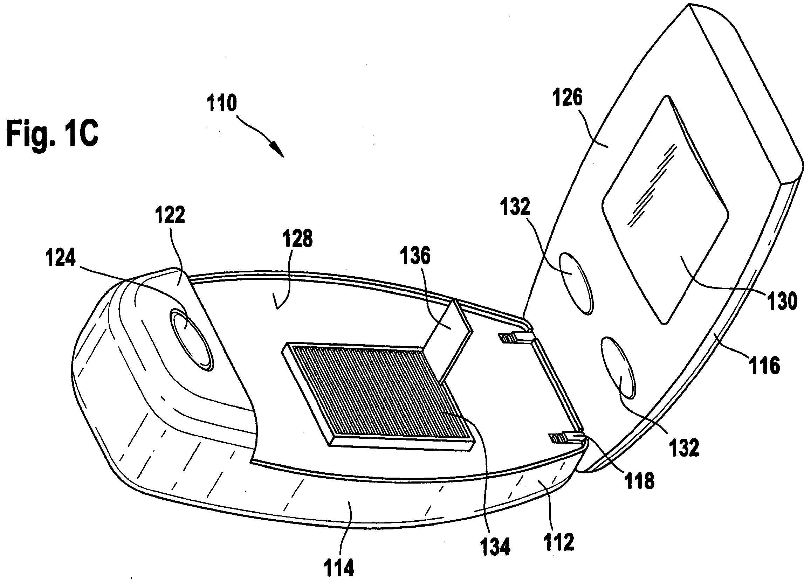

In den Figuren 1A bis 1C ist ein erstes, bevorzugtes Ausführungsbeispiel einer erfindungsgemäßen Testvorrichtung 110 zur Bestimmung einer Blutglukosekonzentration dargestellt. Dabei zeigt Figur 1A die Testvorrichtung 110 in geschlossenem Zustand, Figur 1B die Testvorrichtung 110 während des Öffnens und Figur 1C die Testvorrichtung 110 in geöffnetem Zustand.

Die Testvorrichtung 110 umfasst ein Gehäuse 112 mit einem Gehäuseunterteil 114 und einem Deckel 116 (Gehäuseoberteil). Gehäuseunterteil 114 und Deckel 116 sind entlang eines Scharnieres 118 miteinander verbunden, wobei das Scharnier 118 gleichzeitig zur Kraftübertragung dient. Diese Kraftübertragung gewährleistet, dass die zum Öffnen des Deckels 116 aufgewendete Kraft gleichzeitig im Gehäuseunterteil 114 zur Drehung eines Teststreifens genutzt werden kann (siehe unten).

In dem in Figur 1A dargestellten geschlossenen Zustand weist das Gehäuse 112 eine geschlossene, glatte Oberfläche auf. Deckel 116 und Gehäuseunterteil 114 sind dabei derart von ihrer Form aneinander angepasst, dass lediglich eine schmale Trennfuge 120 entsteht, durch welche im Wesentlichen keine Verunreinigungen in Form von Schmutz und Wasser in das Innere der Testvorrichtung 110 eindringen können. Dabei ist am Gehäuseunterteil 114 eine nach oben weisende Bedienstufe 122 ausgebildet, welche während des Öffnens des Gehäuses 112 beispielsweise mit dem Daumen einer Hand umfasst werden kann. Auf der Bedienstufe 122 ist ein Entriegelungsknopf 124 angeordnet. Dieser Entriegelungsknopf 124 kann rein mechanische Funktion haben, insbesondere indem durch Drücken des Entriegelungsknopfes 124 ein Aufklappen des Gehäuses 112 freigegeben wird. Alternativ oder zusätzlich kann der Entriegelungsknopf 124 auch elektrische Funktion haben, z. B. indem bei Betätigen des Entriegelungsknopfes 124 eine Messelektronik und/oder ein Mikrocomputer in der Testvorrichtung 110 gestartet bzw. initialisiert wird.

Im Inneren des Gehäuses 112 befindet sich auf der Innenseite des Deckels 116 eine Bedienoberfläche 126 und auf der Innenseite des Gehäuseunterteils 114 eine Messoberfläche 128. Die Bedienoberfläche 126 weist ein Anzeigeelement in Form eines Displays 130 sowie mehrere Bedienknöpfe 132 auf. Über die Bedienknöpfe 132 können beispielsweise Messungen gestartet und/oder Menüfunktionen der Testvorrichtung 110 aufgerufen werden. Auf dem Display 130 können beispielsweise Analytkonzentrationen numerisch dargestellt werden. Insgesamt ist die Funktionalität der Testvorrichtung 110 beispielsweise vergleichbar zu bekannten Testvorrichtungen, welche kommerziell erhältlich sind.

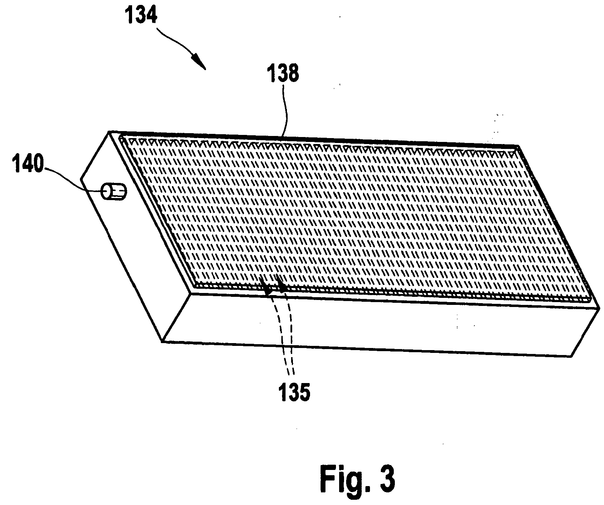

In die Messoberfläche 128 im Gehäuseunterteil 114 ist ein riegelartiges Magazin 134 eingelassen. Das Magazin 134 ist auswechselbar ausgestaltet und weist eine Vielzahl von Kavitäten 135 auf, in welchen Teststreifen 136 parallel zueinander aufgenommen sind. Das Magazin 134, insbesondere die Kavitäten 135, kann zusätzlich ein Trockenmittel (in Figur 3 nicht dargestellt) enthalten, welches die in den Kavitäten 135 aufgenommenen Teststreifen 136 vor Einfluss von Luftfeuchtigkeit schützt. Ein Beispiel eines derartigen Magazins 134 ist in Figur 3 dargestellt. Über jeder Kavität 135 ist eine Membran in Form einer Siegelfolie 138 aufgebracht. Weiterhin weist das Magazin 134 eine Drehachse 140 auf, um welche die einzelnen Teststreifen 136 herum drehbar gelagert sind.

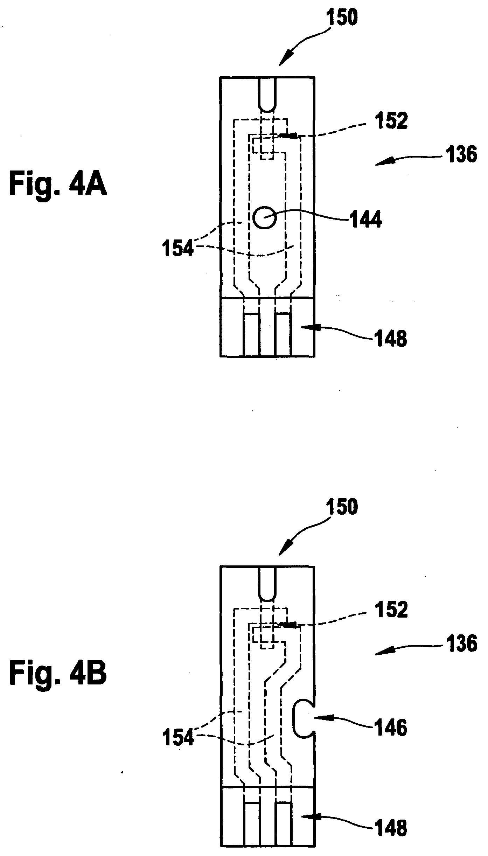

Beim Öffnen des Gehäuses 112 der Testvorrichtung 110 (vgl. Figur 1 B) wird mittels einer Ausgabevorrichtung (nicht dargestellt) jeweils ein Teststreifen 136 um die Drehachse 140 herum gedreht. Dabei wird die Siegelfolie 138 der zugehörigen Kavität 135 durch die Teststreifenkante 142 des Teststreifens 136 aufgebrochen oder durchstoßen. In vollständig aufgeklapptem Zustand des Gehäuses 112 (vgl. Figur 1C) befindet sich der Teststreifen 136 in einer aufrechten Applikations- und Messposition. Alternativ sind auch andere Ausrichtungen des Teststreifens 136 zur Probenapplikation und/oder Messung möglich, beispielsweise eine getrennte Applikationsposition und Messposition. Auch denkbar ist, dass nach dem Aufklappen des Gehäuses 112 zunächst eine Beförderung in eine Applikationsposition und anschließend, nach erfolgter Probenapplikation, in eine Messposition erfolgt, letzteres beispielsweise ausgelöst durch ein erneutes Schließen des Gehäuses 112. Wesentlich ist, dass in der Applikationsposition eine Applikationsstelle 150 des Teststreifens (siehe unten, Figuren 4A und 4B) für den Patienten für eine Probenapplikation zugänglich ist.

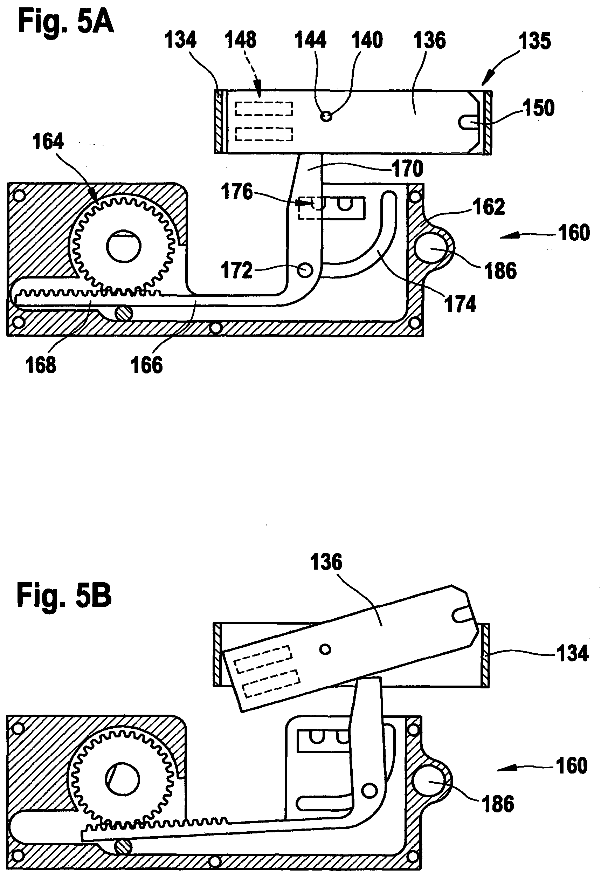

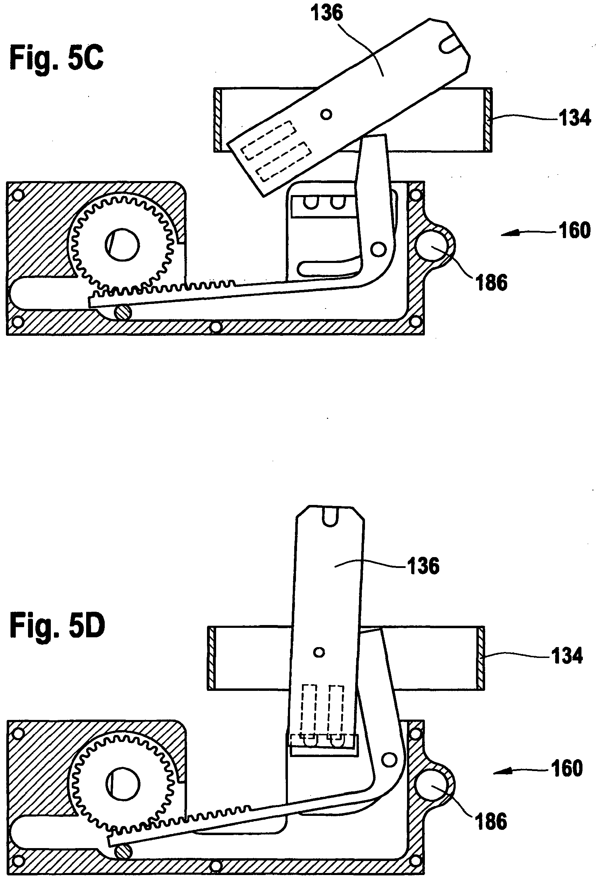

In der in Figur 1C dargestellten Applikations- und Messposition ist der aufgerichtete Teststreifen 136 von Kontakten (nicht dargestellt, siehe z. B. Bezugsziffer 176 in Figur 5A) der Testvorrichtung 110 elektrisch kontaktiert, so dass eine elektrochemische Messung vorgenommen werden kann.

Das in Figur 3 dargestellte Magazin 134 kann als Riegel für beispielsweise 10, 25, 50 oder mehr Teststreifen 136 ausgestaltet sein. Die Begrenzung der Anzahl der Teststreifen 136 liegt in der Größe der Testvorrichtung 110. Für 50 Teststreifen 134 wird eine Magazinlänge von ca. 40 mm benötigt. Teststreifen, welche in einem Magazin 134 gemäß der Ausführung in Figur 3 einsetzbar sind, sind in den Figuren 4A und 4B als Beispiele dargestellt. In dem in Figur 4A dargestellten bevorzugten Ausführungsbeispiel weist der Teststreifen 136 in seiner Mitte ein Loch 144 zur Durchführung der Drehachse 140 (siehe Figur 3) auf. In dem in Figur 4B dargestellten Ausführungsbeispiel des Teststreifens 136 befindet sich eine Einkerbung 146 an einem Rand des Teststreifens 136. Sowohl das Loch 144 als auch die Einkerbung 146 ermöglichen es, dass der Teststreifen 136 um die Drehachse 140 des Magazins 134 von einer (horizontalen) Lagerposition in eine (senkrechte, vgl. Figur 1c) Messposition gedreht wird.

Bei den Teststreifen 136 in den Ausführungsbeispielen gemäß Figur 4A und Figur 4B handelt es sich um elektrochemische Messstreifen zur Blutglukosekonzentrationsmessung gemäß der obigen Beschreibung. Die Teststreifen 136 sind als langgezogene Rechtecke ausgestaltet und weisen an einem Ende Elektrodenkontakte 148 und an einem gegenüberliegenden Ende eine Applikationsstelle 150 zur Applikation eines Blutstropfens auf. Über ein Kapillarsystem 152 wird Blut von der Applikationsstelle 150 ins Innere des Teststreifens 136 befördert. Dort kommt das Blut mit Messelektroden 154 in Kontakt, welche wiederum mit den Elektrodenkontakten 148 verbunden sind. In der (in Figur 1C dargestellten) Messposition sind die Elektrodenkontakte 148 mit entsprechenden Kontakten der Testvorrichtung 110 verbunden, so dass eine elektrochemische Bestimmung der Blutglukosekonzentration durchgeführt werden kann.

Neben der Ausgabevorrichtung zur Beförderung des Teststreifens 136 in die Messposition (vgl. Figuren 1B und 1C) enthält das Gehäuse 112 der Testvorrichtung 110 weiterhin eine elektronische Auswertungsvorrichtung. Diese elektronische Auswertungsvorrichtung dient zur Auswertung der Teststreifen 136 mittels einer elektrochemischen Messung. Derartige Auswertungselektroniken sind dem Fachmann bekannt. Die Auswertungselektronik kann weiterhin auch einen Mikrocomputer umfassen, welcher beispielsweise über die Bedienknöpfe 132 bedient werden kann. Der Mikrocomputer kann entsprechende Auswertungsroutinen steuern und beispielsweise Datenspeicher umfassen.

In dem in den Figuren 1A bis 1C dargestellten Ausführungsbeispiel der Testvorrichtung 110 sind das Display 130 und die Bedienknöpfe 132 in der innenliegenden Bedienoberfläche 126 des Deckels 116 integriert. Wie oben beschrieben, kann beispielsweise ein Öffnen des Gehäuses 112 automatisch ein Einschalten der Testvorrichtung 110 bewirken. Alternativ oder zusätzlich kann ein derartiges Einschalten auch durch den Entriegelungsknopf 124 (welcher auch als "An/Aus-Knopf" realisiert sein kann) erfolgen. Nach Gebrauch der Testvorrichtung 110, also insbesondere nach Durchführung einer Blutglukosekonzentrationsmessung, verschließt der Patient das Gehäuse 112 wieder durch Zuklappen des Deckels 116. Dabei wird der Teststreifen 136 automatisch wieder in seine Kavität 135 zurückgeschwenkt. Alternativ kann der Teststreifen 136 auch manuell entnommen und entsorgt werden. Beim Schließen des Gehäuses 112 kann wahlweise auch die Testvorrichtung 110 automatisch abgeschaltet werden, so dass verhindert wird, dass die Testvorrichtung 110 unbeabsichtigt eingeschaltet bleibt. Dies spart insbesondere elektrische Energie.

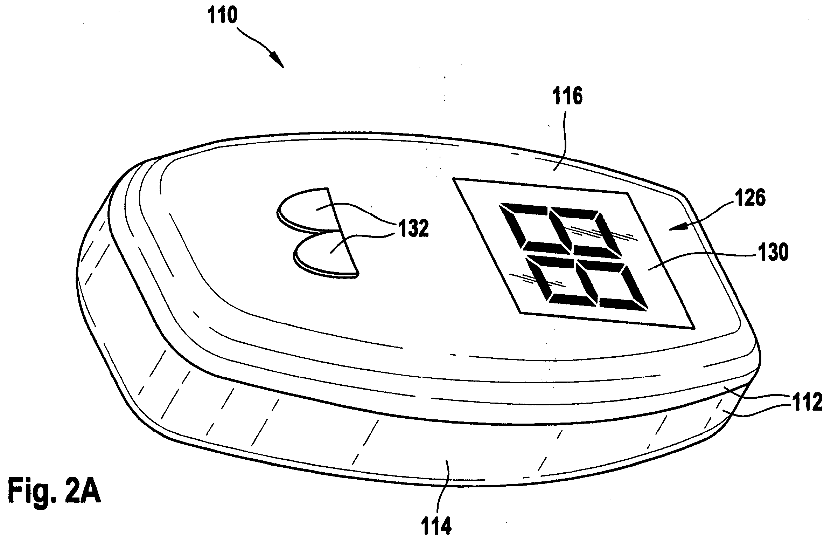

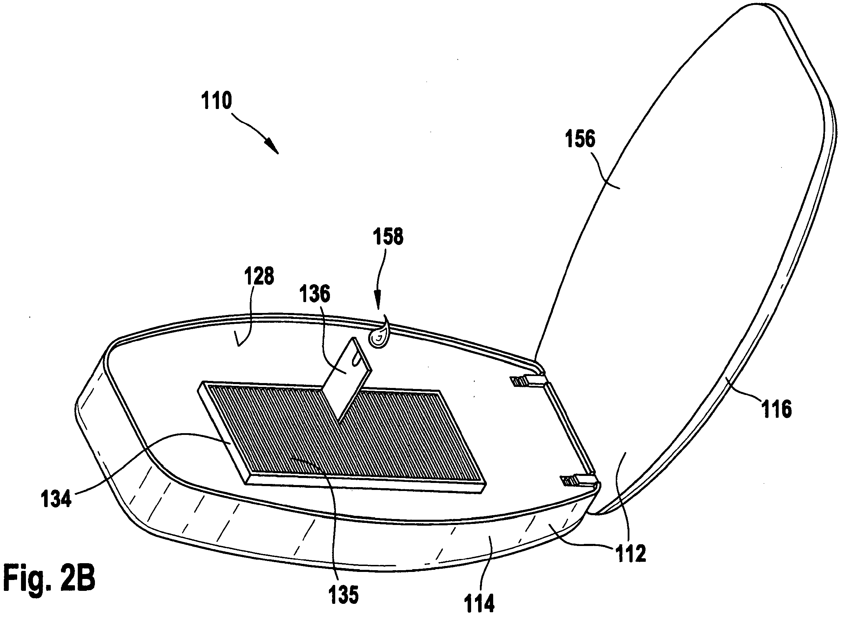

In den Figuren 2A und 2B ist ein zu den Figuren 1A bis 1C alternatives Ausführungsbeispiel 110 dargestellt, welches ähnliche Funktionalität aufweist. Wiederum ist in ein Gehäuse 112 ein Magazin 134, beispielsweise ein Magazin gemäß der Ausführung in Figur 3, eingelassen. Das Magazin 134 weist wiederum Kavitäten 135 auf, in welche elektrochemische Teststreifen 136 eingelassen sind. Die Teststreifen 136 können wiederum ausgestaltet sein analog zu den Ausführungsbeispielen in den Figuren 4A oder 4B.

Im Unterschied zur Ausgestaltung gemäß den Figuren 1A bis 1C weist das Ausführungsbeispiel gemäß den Figuren 2A und 2B zwar eine im Inneren des Gehäuses 112 liegende Messoberfläche 128 auf, jedoch keine innenliegende Bedienoberfläche. Stattdessen ist in diesem Ausführungsbeispiel die Bedienoberfläche 126 mit einem Display 130 und Bedienknöpfen 132 auf der Oberseite des Deckels 116 aufgebracht. Wahlweise kann auch auf der Deckelinnenseite 156 Funktionalität in Form von Anzeigeelementen und/oder Bedienelementen untergebracht sein (nicht dargestellt).

Die Funktion der Testvorrichtung 110 gemäß dem Ausführungsbeispiel in den Figuren 2A und 2B kann beispielsweise dahingehend gestaltet sein, dass ein Patient das Gehäuse 112 öffnet und einen Blutstropfen 158 auf den Teststreifen 136 appliziert. Anschließend wird das Gehäuse 112 wieder verschlossen, wodurch beispielsweise automatisch eine Messung gestartet oder fortgesetzt wird bzw. elektronische Messergebnisse ausgewertet werden, wobei die Ergebnisse wiederum bei geschlossenem Gehäuse 112 vom Display 130 abgelesen werden können.

In den Figuren 5A bis 5E ist schematisch die Ausgabe eines Teststreifens 136 mittels einer erfindungsgemäßen Ausgabevorrichtung 160 beim Öffnen eines Gehäuses 112 einer Testvorrichtung 110 gemäß der Darstellung beispielsweise in den Figuren 1A bis 1C dargestellt. Die Darstellung in den Figuren 5A bis 5E zeigt einen Schnitt parallel zum Scharnier 118 (also senkrecht zur längeren Achse des Gehäuses 112) durch das Gehäuseunterteil 114 der Testvorrichtung 110. Dabei ist das Gehäuse 112 zur Vereinfachung in den Figuren 5A bis 5E nicht dargestellt.

Die Ausgabevorrichtung 160 wirkt mit einem Magazin 134, beispielsweise gemäß der Darstellung in Figur 3, zusammen. In das Magazin 134 sind mehrere Teststreifen 136, in diesem Ausführungsbeispiel Teststreifen 136 gemäß dem Ausführungsbeispiel in Figur 4A, aufgenommen. Die Teststreifen 136 weisen an einem Ende Elektrodenkontakte 148 und am gegenüberliegenden Ende eine Applikationsstelle 150 zur Applikation eines Blutstropfens 158 auf. Weiterhin weisen die Teststreifen 136 jeweils ein Loch 144 auf, durch welches eine Drehachse 140 geführt ist. Das Gehäuseunterteil 114 der Testvorrichtung 110 ist dabei derart ausgestaltet, dass dieses eine Aufnahme zur Lagerung der Enden der Drehachse 140 aufweist. Diese Aufnahme ist in den Figuren 5A bis 5E nicht dargestellt. Die Drehachse 140 stellt somit beispielsweise einen integralen Bestandteil eines Magazins 134 dar, wobei beim Einfügen eines neuen Magazins 134 in ein Gehäuse 112 einer Testvorrichtung 110 die Enden der Drehachse 140 jeweils in entsprechende Aufnahmen im Gehäuse 112 eingerastet oder eingefügt werden. Somit ist jeder Teststreifen 136 um diese Drehachse 140 drehbar gelagert.

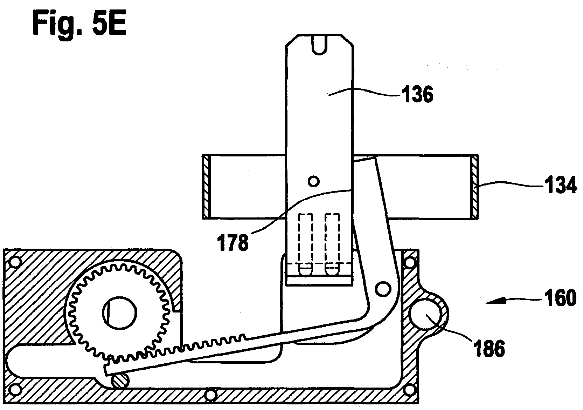

Mittels der Ausgabevorrichtung 160 wird ein Teststreifen 136 von einer Lagerposition (Figur 5A) in eine Messposition (Figur 5E) befördert. Dabei befindet sich der Teststreifen 136 bei geschlossenem Gehäuse 112 (vgl. Darstellung in Figur 1A) in der in Figur 5A dargestellten Lagerposition, wohingegen sich bei geöffnetem Gehäuse 112 (vgl. Figur 1C) der Teststreifen 136 in der in Figur 5E dargestellten Messposition befindet. In der in Figur 5A dargestellten Lagerposition ist der Teststreifen 136 in einer Kavität 135 aufgenommen. In der in Figur 5E dargestellten Messposition hingegen ist der Teststreifen 136 im Vergleich zur Lagerposition gemäß Figur 5A um 90° gedreht, wobei die Elektrodenkontakte 148 nach unten weisen und die Applikationsstelle 150 nach oben weist.

Zu diesem Zweck weist die Ausgabevorrichtung 160 ein Ausgabegehäuse 162 auf, welches unterhalb des Magazins 134 angeordnet ist. Im Ausgabegehäuse 162 ist ein Zahnrad 164 mit einer Drehachse senkrecht zur Zeichenebene drehbar gelagert. Dieses Zahnrad 164 wird mittels eines (nicht dargestellten) Getriebes durch das Scharnier 118 der Testvorrichtung 110 angetrieben und dreht sich beim Öffnen des Gehäuses 112 entgegen dem Uhrzeigersinn.

Weiterhin weist die Ausgabevorrichtung 160 einen Transportarm 166 auf, welche rechtwinklig gebogen ist. In einem Antriebsbereich 168 wirkt der Transportarm 166 mit dem Zahnrad 164 zusammen, derart, dass der Transportarm 166 beim Öffnen des Gehäuses 112 über das Zahnrad 164 in Figur 5A nach rechts bewegt wird.

In der in Figur 5A dargestellten Ruheposition, welche ein geschlossenes Gehäuse 112 der Testvorrichtung 110 repräsentiert, liegt ein nach oben weisender Druckbereich 170 des Transportarms 166 am Unterboden des Magazins 134 an. Im nach oben weisenden Druckbereich 170 ist der Transportarm 166 über einen Stift 172 und ein gebogenes Langloch 174 im Ausgabegehäuse 162 der Ausgabevorrichtung 160 geführt.

Sobald das Gehäuse 112 der Testvorrichtung 110 leicht geöffnet wird, beginnt der Druckbereich 170 des Transportarms 166, in das Magazin 134 einzudringen (siehe Figuren 5B bis 5E). Dabei dringt der Druckbereich 170 jeweils in genau eine Kavität 135 des Magazins 134 ein, wobei genau ein Teststreifen 136 aus dem Magazin 134 herausgedrückt wird. Zu diesem Zweck kann beispielsweise das Magazin 134 oberhalb und unterhalb jeder Kavität 135 eine perforierbare Membran 138 (siehe Figur 3, in den Figuren 5A bis 5E nicht dargestellt) aufweisen. Diese Membran kann von den Kanten des Teststreifens 136 und/oder vom Druckbereich 170 des Transportarms 166 durchstoßen werden. Wird das Gehäuse 112 geöffnet, so wird der Transportarm 166 durch das Zahnrad 164 über den Antriebsbereich 168 in den Figuren 5A bis 5E nach rechts bewegt, wodurch sich der Druckbereich 170 nach oben in das Magazin 134 bewegt. Dabei ist das Magazin 134 derart über der Ausgabevorrichtung 160 gelagert, dass der Druckbereich 170 rechts der Drehachse 140 auf den Teststreifen 136 einwirkt. Dadurch wird der Teststreifen 136 in den Darstellungen gemäß den Figuren 5A bis 5E entgegen dem Uhrzeigersinn gedreht.

Weiterhin weist die Ausgabevorrichtung 160 in diesem Ausführungsbeispiel gemäß den Figuren 5A bis 5E Kontakte 176 zur Kontaktierung des Teststreifens 136 auf. Diese Kontakte 136 sind mit einer Messelektronik der Testvorrichtung 110 verbunden. Diese Messelektronik, welche nicht dargestellt ist, ermöglicht die Auswertung eines Teststreifens 136 unter Ausnutzung von dem Fachmann aus dem Stand der Technik bekannten elektrochemischen Messverfahren. Beispielsweise kann diese Messelektronik eine Elektronik zur Durchführung einer Strom-Spannungsmessung oder einer kapazitiven Messung enthalten. Die Kontakte 176 sind derart an dem Ausgabegehäuse 162 der Ausgabevorrichtung 160 fixiert, dass die Kontakte 176 in der in Figur 5E dargestellten Messposition die Elektrodenkontakte 148 des Teststreifens 136 kontaktieren. Beispielsweise können die Kontakte 176 eine entsprechende Klemmvorrichtung aufweisen, in welche der Teststreifen 136 durch die Ausgabevorrichtung 160, insbesondere durch den Transportarm 166, hineingepresst wird. Auf diese Weise kann ein sicherer und zuverlässiger elektrischer Kontakt zwischen Elektrodenkontakten 148 und Kontakten 176 hergestellt werden.

Aufgrund des gebogenen Langlochs 174 beschreibt der Druckbereich 170 des Transportarms 166 beim Öffnen (Figurenfolge 5A bis 5E) eine gekrümmte Bahn, beispielsweise einen Kreisbogen. Beim Schließen des Gehäuses 112 wird dieser Kreisbogen vom Druckbereich 170 in umgekehrter Richtung durchfahren (Figurenfolge 5E bis 5A). Dabei liegt der Druckbereich 170 mit einer Abflachung 178 an dem Teststreifen 136 an, so dass der Teststreifen 136 beim Schließen des Gehäuses 112 zu einer Drehung im Uhrzeigersinn gezwungen wird. Dadurch wird nach Benutzung des Teststreifens 136 der benutzte Teststreifen 136 wieder zurück von der Messposition (Figur 5E) in die Lagerposition (Figur 5A) überführt.

Die in den Figuren 5A bis 5E dargestellte Ausgabevorrichtung 160 stellt einen Mechanismus zur Ausgabe genau eines Teststreifens 136 aus dem Magazin 134 zur Verfügung. Je nach Positionierung der Ausgabevorrichtung 160 unterhalb einer bestimmten Kavität 135 des Magazins 134 wird gerade derjenige Teststreifen 136 in die Messposition (siehe Figur 5E) überführt, welcher sich in der entsprechenden Kavität 135 befindet.

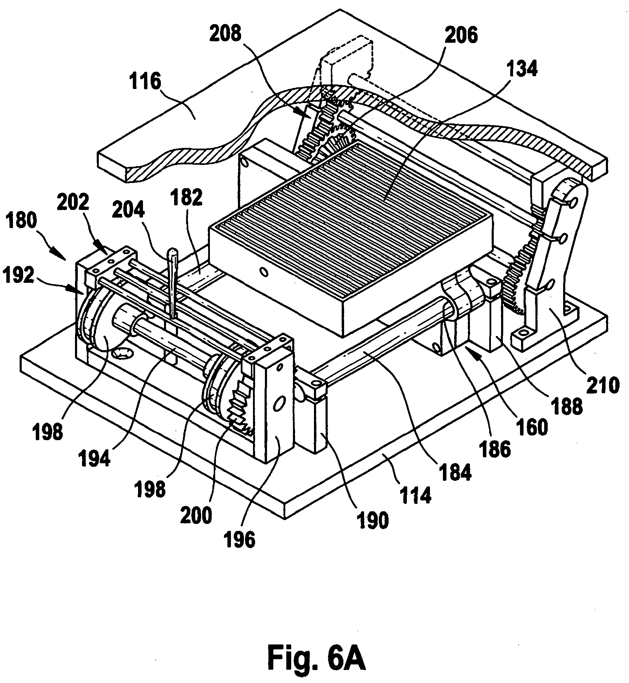

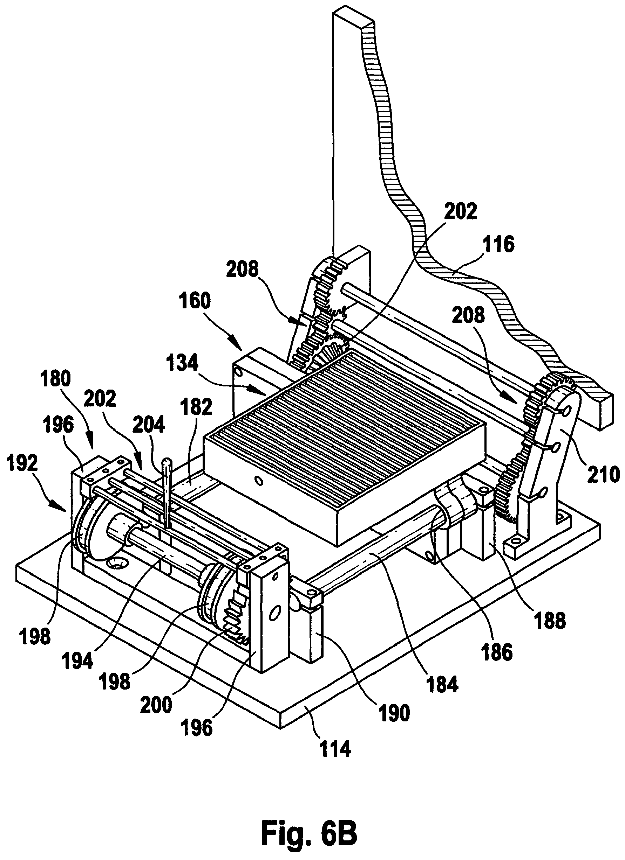

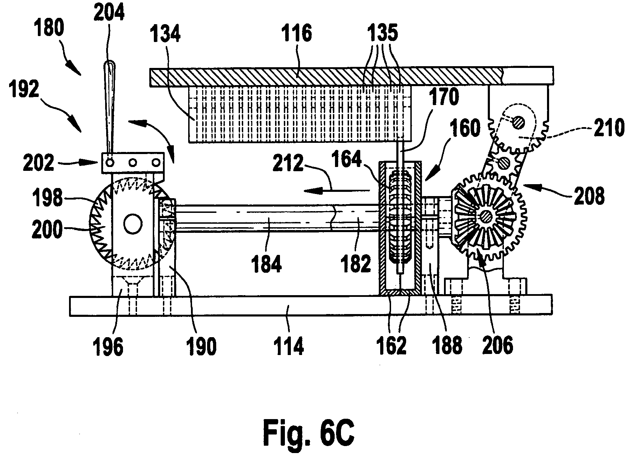

Dabei kann die Testvorrichtung 110 einen Mechanismus umfassen, welcher die Anwahl einer bestimmten Kavität 135 des Magazins 134 umfasst. Ein Beispiel einer derartigen Auswahlvorrichtung 180 ist in den Figuren 6A bis 6D in verschiedenen Ansichten schematisch dargestellt. Dabei zeigt Figur 6A eine perspektivische Teilansicht mit geschlossenem Deckel 116, Figur 6B eine perspektivische Teilansicht mit geöffnetem Deckel 116, Figur 6C eine Seitenansicht mit geschlossenem Deckel 116 und Figur 6C eine Seitenansicht mit geöffnetem Deckel. Die Figuren 6A bis 6C sollen in Zusammenschau erläutert werden.

Die Ausgabevorrichtung 160 gemäß dem oben in den Figuren 5A bis 5E beschriebenen Ausführungsbeispiel ist auf zwei Wellen 182, 184 gelagert, nämlich einer Antriebswelle 182 und einer Gleitwelle 184, welche Bestandteile der Auswahlvorrichtung 180 sind. Die Antriebswelle 182 ist mit dem Zahnrad 164 der Ausgabevorrichtung 160 verbunden und treibt dieses an. Die Gleitwelle 184 durchsetzt eine entsprechende Gleitwellenbohrung 186 im Ausgabegehäuse 162, so dass die Ausgabevorrichtung 160 auf der Gleitwelle 184 gleiten kann. Eine deckelseitige (also in den Figuren 6A bis 6D rechte) Anschlagsposition der Ausgabevorrichtung 160 ist durch einen mit dem Gehäuseunterteil 114 fest verbundenen Anschlag 188 vorgegeben, welcher auch gleichzeitig das deckelseitige Ende der Gleitwelle 184 haltert. Das entgegengesetzte Ende der Gleitwelle 184 ist in einer Halterung 190 gehaltert.

Weiterhin weist die Auswahlvorrichtung 180 eine Antriebseinheit 192 auf. Die Antriebseinheit 192 weist ihrerseits eine Querwelle 194 auf, welche an zwei Seitenhalterungen 196 gelagert ist und welche sich senkrecht zu den Wellen 182, 184 erstreckt. Auf der Querwelle 194 sind zwei Trommeln 198 und ein Unruh 200 befestigt. Die Querwelle 194 ist durch eine (nicht dargestellte) Feder vorgespannt. Weiterhin verfügt die Antriebseinheit 192 über eine Wippe 202 mit einem Auslösehebel 204. Dieser Auslösehebel 204 ist mit dem Entriegelungsknopf 124 (vgl. z. B. Figur 1A) verbunden. Wird der Auslösehebel 204 betätigt, so gibt die Wippe 202 das Unruh 200 kurzfristig frei, so dass sich dieses, getrieben durch die Feder, gerade um eine Raste (entsprechend einem Zahn auf dem Unruh 200) weiterbewegen kann.
Auf den Trommeln 198 ist ein (in den Figuren nicht dargestelltes) Federband aufgewickelt, welches wiederum mit der Ausgabevorrichtung 160 verbunden ist. Dreht sich das Unruh 200, so drehen sich auch die Trommeln 198, wodurch das Federband auf die Trommeln aufgewickelt wird. Dadurch wird die Ausgabevorrichtung 160 in der Bewegungsrichtung 212, also in den Figuren 6A bis 6D nach links, bewegt.

Pro Raste des Unruhs 200, also pro Betätigung des Auslösehebels 204, erfolgt eine definierte Bewegung der Ausgabevorrichtung 160 um einen Schritt. Über der Ausgabevorrichtung 160 ist, wie oben beschrieben, das Magazin 134 angeordnet. Das Unruh 200 und die Übersetzung durch die Trommeln 198 sind dabei so bemessen, dass ein Bewegungsschritt gerade der Positionierung um eine Kavität 135 vorwärts bedeutet. Somit wird bei jeder Raste die Ausgabevorrichtung 160 um eine Kavität 135 weiterbefördert.

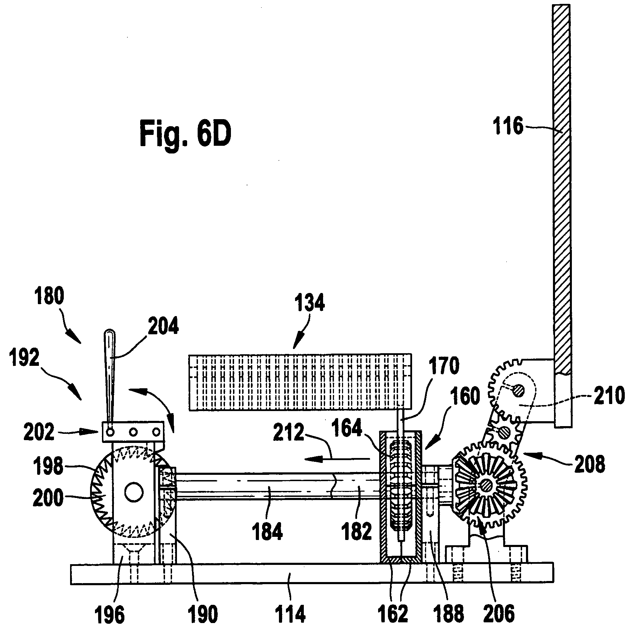

Die Antriebswelle 182 ist über ein Winkelgetriebe 206 mit einem Zahnradgetriebe 208 des Deckels 116 verbunden (siehe Fig. 6C und 6D). Das Zahnradgetriebe 208 wiederum ist an einer mit dem Gehäuseunterteil 114 fest verbundenen Deckelhalterung 210 gelagert, so dass eine Aufklappbewegung des Deckels 116 relativ zum Gehäuseunterteil 114 ermöglicht ist. Gleichzeitig ist der Deckel 116 relativ zum Gehäuseunterteil 114 durch eine (nicht dargestellte) Feder vorgespannt in Richtung einer Aufklappbewegung. Im geschlossenen Zustand des Gehäuses 112 (Figuren 6A und 6C) wird der Deckel 116 durch eine Verriegelung (nicht dargestellt) festgehalten, welche ein Aufklappen des Deckels 116 infolge der verhindert. Erst durch Betätigen des Entriegelungsknopfes 124 wird der Deckel 116 freigegeben und kann, getrieben durch die Federkraft, aufklappen. Über das Winkelgetriebe 206 wird jedoch beim Aufklappen des Deckels 116 die Antriebswelle 182 gedreht, welche wiederum das Zahnrad 164 der Ausgabevorrichtung 160 antreibt, wodurch über den Transportarm 166 ein Teststreifen 136 aus dem Magazin 134 ausgegeben wird.

Insgesamt gestaltet sich ein Ausgabevorgang eines Teststreifens 136 aus dem Magazin 134 also nach den folgenden Schritten. Ein Benutzer betätigt den Entriegelungsknopf 124 des Gehäuses 112. Dabei wird über den Auslösehebel 204 und das Unruh 200 die Ausgabevorrichtung 160 um genau eine Kavität 135 weiterpositioniert. Gleichzeitig wird durch Betätigen des Entriegelungsknopfes 124 der Deckel 116 freigegeben und klappt, getrieben durch Federkraft, auf. Über die Antriebswelle 182 wird dabei das Zahnrad 164 der Ausgabevorrichtung 160 betätigt, wodurch wiederum die Ausgabevorrichtung 160 mittels des Transportarms 166 genau einen Teststreifen 136 in die in Figur 5E dargestellte Applikations- und Messposition befördert, so dass eine Messung durchgeführt werden kann.

### Bezugszeichen

- 110: Testvorrichtung
- 112: Gehäuse
- 114: Gehäuseunterteil
- 116: Deckel
- 118: Scharnier
- 120: Trennfuge
- 122: Bedienstufe
- 124: Entriegelungsknopf
- 126: Bedienoberfläche
- 128: Messoberfläche
- 130: Display
- 132: Bedienknöpfe
- 134: Magazin
- 135: Kavitäten
- 136: Teststreifen
- 138: Siegelfolie
- 140: Drehachse
- 142: Teststreifenkante
- 144: Loch
- 146: Einkerbung
- 148: Elektrodenkontakte
- 150: Applikationsstelle
- 152: Kapillarsystem
- 154: Messelektroden
- 156: Deckelinnenseite
- 158: Blutstropfen
- 160: Ausgabevorrichtung
- 162: Ausgabegehäuse
- 164: Zahnrad
- 166: Transportarm
- 168: Antriebsbereich
- 170: Druckbereich
- 172: Führungsstift
- 174: gebogenes Langloch
- 176: Kontakte
- 178: Abflachung
- 180: Auswahlvorrichtung
- 182: Antriebswelle
- 184: Gleitwelle
- 186: Gleitwellenbohrung
- 188: Anschlag
- 190: Halterung
- 192: Antriebseinheit
- 194: Querwelle
- 196: Seitenhalterungen
- 198: Trommeln
- 200: Unruh
- 202: Wippe
- 204: Auslösehebel
- 206: Winkelgetriebe
- 208: Zahnradgetriebe
- 210: Deckelhalterung
- 212: Bewegungsrichtung

## Patentansprüche

1. Testvorrichtung (110) zur Bestimmung mindestens einer Analytkonzentration in einer Probe mittels Testelementen (136), mit
- einem Gehäuse (112), wobei das Gehäuse (112) einen geschlossenen Zustand und einen geöffneten Zustand aufweist;
- einer Lagervorrichtung (134) zur Aufnahme von Testelementen (136), wobei die Lagervorrichtung (134) mindestens eine Lagerposition für Testelemente (136) aufweist;
- einer Messvorrichtung, wobei die Messvorrichtung ausgestaltet ist, um mittels der Testelemente (136) die mindestens eine Analytkonzentration zu bestimmen; und
- einer Ausgabevorrichtung (140, 160), wobei die Ausgabevorrichtung (140, 160) Mittel (140, 166) zur Beförderung der Testelemente (136) von der mindestens einen Lagerposition in mindestens eine Messposition bei Öffnen des Gehäuses (112) aufweist,
**dadurch gekennzeichnet, dass**
- die Testelemente (136) als streifenförmige Testelemente (136) ausgestaltet sind, wobei die Lagervorrichtung (134) ein Magazin (134) aufweist, wobei das Magazin (134) eine Vielzahl von Kavitäten (135) aufweist, wobei in den Kavitäten (135) streifenförmige Testelemente (136) parallel zueinander jeweils einzeln aufgenommen sind.

2. Testvorrichtung (110) gemäß dem vorhergehenden Anspruch, **dadurch gekennzeichnet, dass** das Gehäuse (112) aufklappbar ist.

3. Testvorrichtung (110) gemäß einem der beiden vorhergehenden Ansprüche, **dadurch gekennzeichnet, dass** die Ausgabevorrichtung (140, 160) eine Drehachse (140) zur Drehung der Testelemente (136) von der mindestens einen Lagerposition in die mindestens eine Messposition aufweist.

4. Testvorrichtung (110) gemäß einem der vorhergehenden Ansprüche, **dadurch gekennzeichnet, dass** die Lagervorrichtung (134) mindestens eine von den Testelementen (136) perforierbare Membran (138) zum Schutz der Testelemente (136) gegen Luftfeuchtigkeit und Schmutz aufweist.

5. Testvorrichtung (110) gemäß einem der vorhergehenden Ansprüche, **dadurch gekennzeichnet, dass** die Lagervorrichtung (134) zusätzlich mindestens ein Trockenmittel zur Verringerung der Luftfeuchtigkeit aufweist.

6. Testvorrichtung (110) gemäß dem vorhergehenden Anspruch, wobei das Trockenmittel in mindestens eines der folgenden Elemente eingebracht ist: in mindestens eine Kavität (135) in der Lagervorrichtung (134); in mindestens ein Testelement (136); in mindestens ein Magazin (134).

7. Testvorrichtung (110) gemäß einem der vorhergehenden Ansprüche, **dadurch gekennzeichnet, dass** die mindestens eine Messposition Kontakte (176) zur Kontaktierung der Testelemente (136) aufweist.

8. Testvorrichtung (110) gemäß einem der vorhergehenden Ansprüche, **dadurch gekennzeichnet, dass** die Ausgabevorrichtung (140, 160) Mittel (140, 166) zur Beförderung der Testelemente (136) von der mindestens einen Messposition in die mindestens eine Lagerposition beim Schließen des Gehäuses (112) aufweist.

9. Testvorrichtung (110) gemäß einem der vorhergehenden Ansprüche, mit zusätzlich einer Auswahlvorrichtung (180), wobei die Auswahlvorrichtung (180) ausgestaltet ist, um bei jedem Öffnen des Gehäuses (112) ein unbenutztes Testelement (136) auszuwählen.

10. Testvorrichtung (110) gemäß einem der vorhergehenden Ansprüche mit zusätzlich einem Entkopplungsmechanismus, wobei der Entkopplungsmechanismus ausgestaltet ist, um bei einer Betätigung des Entkopplungsmechanismus durch einen Benutzer eine Beförderung der Testelemente (136) durch die Ausgabevorrichtung (140, 160) von der mindestens einen Lagerposition in die mindestens eine Messposition beim Öffnen des Gehäuses (112) zu verhindern.

11. Testvorrichtung (110) gemäß einem der vorhergehenden Ansprüche, **dadurch gekennzeichnet, dass** die Messvorrichtung Mittel zur Durchführung mindestens einer der folgenden Messungen aufweist: eine Glukosemessung, insbesondere einer Blutglukosemessung; eine Cholesterinmessung; eine Koagulationsmessung; eine immunologische Messung.

12. Testvorrichtung (110) gemäß Anspruch 3, wobei das Magazin (134) als auswechselbares Magazin (134) ausgestaltet ist.

13. Magazin (134) mit Testelementen (136) zur Bestimmung mindestens einer Analytkonzentration in einer Probe, wobei das Magazin (134) ausgestaltet ist, um in einer Testvorrichtung (110) gemäß Anspruch 12 verwendet zu werden, wobei die Testelemente (136) als streifenförmige Testelemente (136) ausgestaltet sind und mindestens eine Verbindungsvorrichtung (144; 146) zur Verbindung des Testelements (136) mit der Ausgabevorrichtung (140, 160) aufweisen, wobei das Magazin (134) eine Vielzahl von gestapelten Kavitäten (135) aufweist, wobei in den Kavitäten (135) streifenförmige Testelemente (136) parallel zueinander jeweils einzeln aufgenommen sind, **dadurch gekennzeichnet, dass** das Magazin (134) eine Drehachse (140) aufweist, um welche die einzelnen Testelemente (136) von der mindestens einen Lagerposition in die mindestens eine Messposition drehbar gelagert sind, wobei die Verbindungsvorrichtung (144; 146) mindestens eine Öffnung (144; 146) aufweist, durch welche die Drehachse (140) geführt ist.

14. Magazin (134) gemäß dem vorhergehenden Anspruch, **dadurch gekennzeichnet, dass** die Testelemente (136) Testelemente (136) zur elektrochemischen Bestimmung der mindestens einen Analytkonzentration sind.

15. Magazin (134) gemäß einem der beiden vorhergehenden Ansprüche, **dadurch gekennzeichnet, dass** die Testelemente (136) ein Kapillarsystem (152) zur Beförderung einer flüssigen Probe von einer Applikationsstelle (150) zu einer Messstelle (154) aufweisen.

## Claims

1. Test device (110) for determining at least one analyte concentration in a sample by means of test elements (136), with
- a housing (112), said housing (112) having a closed state and an opened state;
- a storage device (134) for receiving test elements (136), said storage device (134) having at least one storage position for test elements (136);
- a measuring device, said measuring device being designed to determine the at least one analyte concentration by means of the test elements (136); and
- a dispensing device (140, 160), said dispensing device (140, 160) comprising means (140, 166) for conveying the test elements (136) from the at least one storage position to at least one measurement position during opening of the housing (112),
**characterized in that**
- the test elements (136) are designed as strip-shaped test elements (136), and the storage device (134) comprises a magazine (134), said magazine (134) comprising a large number of cavities (135), with strip-shaped test elements (136) being received parallel to one another in each case individually in said cavities (135).

2. Test device (110) according to the preceding claim, **characterized in that** the housing (112) can be folded open.

3. Test device (110) according to one of the two preceding claims, **characterized in that** the dispensing device (140, 160) has a rotation axle (140) for rotating the test elements (136) from the at least one storage position to the at least one measurement position.

4. Test device (110) according to one of the preceding claims, **characterized in that** the storage device (134) has at least one membrane (138) that can be perforated by the test elements (136) and that protects the test elements (136) from air moisture and dirt.

5. Test device (110) according to one of the preceding claims, **characterized in that** the storage device (134) additionally has at least one desiccant for reducing the air moisture.

6. Test device (110) according to the preceding claim, the desiccant being incorporated in at least one of the following elements: in at least one cavity (135) in the storage device (134); in at least one test element (136); in at least one magazine (134).

7. Test device (110) according to one of the preceding claims, **characterized in that** the at least one measurement position has contacts (176) for contacting the test elements (136).

8. Test device (110) according to one of the preceding claims, **characterized in that** the dispensing device (140, 160) comprises means (140, 166) for conveying the test elements (136) from the at least one measurement position to the at least one storage position during closure of the housing (112) is closed.

9. Test device (110) according to one of the preceding claims, additionally with a selector device (180), said selector device (180) being designed to select an unused test element (136) each time the housing (112) is opened.

10. Test device (110) according to one of the preceding claims, additionally with a decoupling mechanism, said decoupling mechanism being designed in such a way that, when the decoupling mechanism is actuated by a user, it prevents the dispensing device (140, 160) from conveying the test elements (136) from the at least one storage position to the at least one measurement position during opening of the housing (112).

11. Test device (110) according to one of the preceding claims, **characterized in that** the measuring device has means for carrying out at least one of the following measurements: a glucose measurement, in particular a blood glucose measurement; a cholesterol measurement; a coagulation measurement; an immunology measurement.

12. Test device (110) according to Claim 3, the magazine (134) being designed as an exchangeable magazine (134).

13. Magazine (134) with test elements (136) for determining at least one analyte concentration in a sample, said magazine (134) being designed to be used in a test device (110) according to Claim 12, the test elements (136) being designed as strip-shaped test elements (136) and having at least one connecting device (144; 146) for connecting the test element (136) to the dispensing device (140, 160), said magazine (134) comprising a large number of stacked cavities (135), and strip-shaped test elements (136) being received parallel to one another in each case individually in said cavities (135), **characterized in that** the magazine (134) has a rotation axle (140), the individual test elements (136) being mounted so as to be rotatable about said rotation axle from the at least one storage position into the at least one measurement position, the connecting device (144; 146) having at least one opening (144; 146) through which the rotation axle (140) passes.

14. Magazine (134) according to the preceding claim, **characterized in that** the test elements (136) are test elements (136) for electrochemical determination of the at least one analyte concentration.

15. Magazine (134) according to one of the two preceding claims, **characterized in that** the test elements (136) have a capillary system (152) for conveying a liquid sample from an application site (150) to a measurement site (154).

## Revendications

1. Dispositif de test (110) pour la détermination d'au moins une concentration d'analyte dans un échantillon au moyen d'éléments d'analyse (136), avec
- un boîtier (112), dans lequel le boîtier (112) présente un état fermé et un état ouvert;
- un dispositif de stockage (134) destiné à contenir des éléments d'analyse (136), dans lequel le dispositif de stockage (134) présente au moins une position de stockage pour des éléments d'analyse (136);
- un dispositif de mesure, dans lequel le dispositif de mesure est conçu pour déterminer ladite au moins une concentration d'analyte au moyen des éléments d'analyse (136); et
- un dispositif de délivrance (140, 160), dans lequel le dispositif de délivrance (140, 160) présente des moyens (140, 166) pour le transport des éléments d'analyse (136) de ladite au moins une position de stockage à au moins une position de mesure lors de l'ouverture du boîtier (112),
**caractérisé en ce que**
- les éléments d'analyse (136) sont constitués par des éléments d'analyse en forme de bandes (136), dans lequel le dispositif de stockage (134) présente un magasin (134), dans lequel le magasin (134) présente une multiplicité de cavités (135), dans lequel des éléments d'analyse en forme de bandes (136) sont logés chaque fois individuellement, parallèlement l'un à l'autre dans les cavités (135).

2. Dispositif de test (110) selon la revendication précédente, **caractérisé en ce que** le boîtier (112) peut être ouvert.

3. Dispositif de test (110) selon l'une des deux revendications précédentes, **caractérisé en ce que** le dispositif de délivrance (140, 160) présente un axe de rotation (140) pour la rotation des éléments d'analyse (136) de ladite au moins une position de stockage à ladite au moins une position de mesure.

4. Dispositif de test (110) selon l'une quelconque des revendications précédentes, **caractérisé en ce que** le dispositif de stockage (134) présente au moins une membrane (138) pouvant être perforée par les éléments d'analyse (136) pour la protection des éléments d'analyse (136) contre l'humidité de l'air et l'encrassement.

5. Dispositif de test (110) selon l'une quelconque des revendications précédentes, **caractérisé en ce que** le dispositif de stockage (134) présente en outre au moins un agent de déshydratation pour diminuer l'humidité de l'air.

6. Dispositif de test (110) selon la revendication précédente, **caractérisé en ce que** l'agent de déshydratation est introduit dans au moins un des éléments suivants:
dans au moins une cavité (135) dans le dispositif de stockage (134); dans au moins un élément d'analyse (136); dans au moins un magasin (134).

7. Dispositif de test (110) selon l'une quelconque des revendications précédentes, **caractérisé en ce que** ladite au moins une position de mesure présente des contacts (176) pour la mise en contact des éléments d'analyse (136).

8. Dispositif de test (110) selon l'une quelconque des revendications précédentes, **caractérisé en ce que** le dispositif de délivrance (140, 160) présente des moyens (140, 166) pour le transport des éléments d'analyse (136) de ladite au moins une position de mesure à ladite au moins une position de stockage lors de la fermeture du boîtier (112).

9. Dispositif de test (110) selon l'une quelconque des revendications précédentes, avec en outre un dispositif de sélection (180), dans lequel le dispositif de sélection (180) est conçu pour sélectionner un élément d'analyse inutilisé (136) à chaque ouverture du boîtier (112).

10. Dispositif de test (110) selon l'une quelconque des revendications précédentes, avec en outre un mécanisme de découplage, dans lequel le mécanisme de découplage est conçu pour empêcher, lors d'un actionnement du mécanisme de découplage par un utilisateur, un transport des éléments d'analyse (136) par le dispositif de délivrance (140, 160) de ladite au moins une position de stockage à ladite au moins une position de mesure lors de l'ouverture du boîtier (112).

11. Dispositif de test (110) selon l'une quelconque des revendications précédentes, **caractérisé en ce que** le dispositif de mesure présente des moyens pour effectuer au moins une des mesures suivantes: une mesure de glucose, en particulier une mesure du glucose du sang; une mesure de cholestérol; une mesure de coagulation; une mesure immunologique.

12. Dispositif de test (110) selon la revendication 3, **caractérisé en ce que** le magasin (134) est constitué par un magasin interchangeable (134).

13. Magasin (134) avec des éléments d'analyse (136) pour la détermination d'au moins une concentration d'analyte dans un échantillon, dans lequel le magasin (134) est conçu pour être utilisé dans un dispositif de test (110) selon la revendication 12, dans lequel les éléments d'analyse (136) sont constitués par des éléments d'analyse en forme de bandes (136) et présentent au moins un dispositif de liaison (144; 146) pour la liaison de l'élément d'analyse (136) au dispositif de délivrance (140, 160), dans lequel le magasin (134) présente une multiplicité de cavités empilées (135), dans lequel des éléments d'analyse en forme de bandes (136) sont logés chaque fois individuellement, parallèlement l'un à l'autre dans les cavités (135), **caractérisé en ce que** le magasin (134) présente un axe de rotation (140), autour duquel les éléments d'analyse individuels (136) peuvent tourner de ladite au moins une position de stockage à ladite au moins une position de mesure, dans lequel le dispositif de liaison (144; 146) présente au moins une ouverture (144; 146), à travers laquelle l'axe de rotation (140) est mené.

14. Magasin (134) selon la revendication précédente, **caractérisé en ce que** les éléments d'analyse (136) sont des éléments d'analyse (136) destinés à la détermination électrochimique de ladite au moins une concentration d'analyte.

15. Magasin (134) selon l'une des deux revendications précédentes, caractérisé en que les éléments d'analyse (136) présentent un système capillaire (152) pour le transport d'un échantillon liquide depuis un point d'application (150) jusqu'à un point de mesure (154).
